# EUROPÄISCHE PATENTSCHRIFT

(11) **EP 0 768 332 B1**
(45) Veröffentlichungstag und Bekanntmachung des Hinweises auf die Patenterteilung: **28.03.2001**
(21) Anmeldenummer: 95810637.9
(22) Anmeldetag: 11.10.1995
(51) Int. Cl.: C08H 1/06, A61L 27/00

(54) **Verfahren zu einer photooxidativen Behandlung von kollagenhaltigen Geweben**
Process for photooxidative treatment of collagen-containing tissues
Procédé de traitement photooxydatif de tissus contenant du collagène

(43) Veröffentlichungstag der Anmeldung: 16.04.1997
(73) Patentinhaber: Sulzer Markets and Technology AG, 8401 Winterthur (CH)
(72) Erfinder: Pendl, Roger, Dr., CH-8357 Guntershausen (CH); Müller-Glauser, Werner, Dr., CH-8542 Wiesendangen (CH); Bittmann, Peter, Dr., CH-8057 Zürich (CH)
(74) Vertreter: Sulzer Management AG

(56) Entgegenhaltungen:
- EP-A- 0 411 925
- BIOCHIMICA ET BIOPHYSICA ACTA, Bd. 1206, Nr. 2, 1994 NL, Seiten 225-230, RAMSHAW ET AL. 'Methylene blue sensitized photooxidation of collagen fibrils'
- JOURNAL OF BIOMEDICAL MATERIALS RESEARCH, Bd. 28, Nr. 5, 1994 US, Seiten 611-618, MOORE M. A. ET AL. 'Stabilization of pericardial tissue by dye-mediated photooxidation'

## Beschreibung

Die Erfindung betrifft ein Verfahren zu einer photooxidativen Behandlung von kollagenhaltigen Geweben gemäss Oberbegriff von Anspruch 1. Kollagenhaltige Gewebe sind beispielsweise Knorpelgewebe und Gewebe von Bändern oder Sehnen. Die Erfindung bezieht sich auch auf derartige Gewebe, die nach dem erfindungsgemässen Verfahren präpariert worden sind.

Eine photooxidative Behandlung von kollagenhaltigem Gewebe (insbesondere Herzbeutelgewebe) ist aus der US-5 147 514 bekannt. Bei diesem Verfahren reagieren Tyrosin-, Tryptophan- und/oder Histidinreste von Kollagenfasern unter der katalytischen Wirkung von durch Licht aktivierten Farbstoffmolekülen mit Sauerstoff derart, dass sich Verbindungen zwischen Kollagenfasern ausbilden. Als wirksame Farbstoffe werden Methylenblau, Methylengrün, Bengalrosa, Riboflavin, Proflavin, Fluoreszein, Eosin und Pyridoxal-5-phosphat genannt.

Knorpelgewebe ist - abgesehen von vereinzelt vorliegenden Chondrocyten - aus einem Netzwerk von Kollagenfasern und hochmolekularem Proteoglykan aufgebaut. Zu rund 75% besteht das Gewebe aus Wasser. Aufgrund dieser Zusammensetzung ist der Knorpel ein weichelastisches, offenporiges und permeables Gewebe. Wasser, das sich im Gewebe befindet, ist leicht mit Wasser der Umgebung austauschbar. Ein Austausch von Wasser oder von in dem Wasser gelösten Stoffen kann ausser durch Diffusion auch durch eine Konvektion erfolgen, die mit Volumenänderungen durch Komprimieren und Expandieren des Gewebes einhergeht. Für eine Konditionierung des Knorpelgewebes und dessen Beladung mit einem Farbstoff ist die gute Austauschfähigkeit eine Voraussetzung. Diese Feststellungen gelten auch für Sehnen und Bänder.

Das bekannte Verfahren wird bei der photooxidativen Behandlung von aus Herzbeuteln gewonnenen Gewebe angewendet, das für die Herstellung von implantierbaren Herzklappen vorgesehen ist. Dieses Gewebe ist flächenartig ausgebildet und dünnwandig - rund 0,5 mm dick - und kann von beiden Seiten her belichtet werden.

Dass die Kollagenfasern durch die photooxidative Behandlung verändert werden, lässt sich mit Kollagenase nachweisen. Dieses Enzym löst die Fasern des unbehandelten Gewebes in Teile auf, mit der Folge, dass der mechanische Zusammenhalt des Gewebes verloren geht. Nach einer photooxidativen Behandlung ist diese "Verdauung" durch Kollagenase nicht mehr in gleichem Ausmass wirksam.

Die Kollagenfasern werden durch die photooxidative Behandlung teilweise miteinander vernetzt, wodurch das Gewebe eine grössere mechanische Beanspruchbarkeit gewinnt. Ausserdem werden unter der katalytischen Wirkung des Farbstoffs Kollagenfasern in der Weise photooxidativ verändert, dass bei Verwendung des behandelten Gewebes als Implantat immunologische Abwehrreaktionen unterbleiben oder nur schwach ausgeprägt sind.

Für die chirurgische Behandlung von Kniegelenken besteht der Wunsch - analog zu den Herzklappen aus Herzbeuteln-Knorpelpräparate als Implantate verwenden zu können. Gelenkknorpel sind 2 bis 3 mm dick und auf Knochen aufgewachsen. Für die Nutzung als Implantat soll das Knorpelgewebe zusammen mit einem Stück zugehörigen Knochenmaterials verwendet werden. Dies bedeutet, dass das Gewebe nur von der Seite der freiliegenden Oberfläche her belichtet werden kann. Somit muss das Licht bis 3 mm tief in das Gewebe eindringen können. Wie sich jedoch experimentell gezeigt hat, ist die Lichtdurchlässigkeit ziemlich schlecht.

Es ist daher Aufgabe der Erfindung, ein Verfahren zu schaffen, das eine photooxidative Behandlung des Kollagennetzwerks trotz schlechter Lichtdurchlässigkeit des natürlichen Knorpelgewebes ermöglicht. Diese Aufgabe wird mit den in Anspruch 1 genannten Massnahmen gelöst.

Die Kollagenfasern und das Proteoglykan bilden eine Vielzahl von Streuzentren, aufgrund derer das Licht nur in die Randbereiche des Knorpelgewebes wirksam eindringen kann. Die Lichtstreuung beruht darauf, dass sich der Brechungsindex bei den Streuzentren lokal stark ändert. Die erfindungsgemässe Lehre geht daher von der Idee aus, den Brechungsindex des Wassers, das den Raum zwischen den Streuzentren füllt, durch Zusetzen geeigneter Stoffe zu erhöhen, so dass der Brechungsindex eine Nivellierung seiner Werte im Gewebe erfährt. Zusatzstoffe, die sich zu diesem Zweck verwenden lassen, sind beispielsweise Diethylenglycol (Brechungsindex n = 1,447 bei 20°C) oder Glycerin (n = 1,474).

Bei dem erfindungsgemässen Verfahren zu einer photooxidativen Behandlung von kollagenhaltigen Geweben wird das Gewebe vor einer Bestrahlung mit Licht mittels einer wässrigen Konditionierungslösung vorbehandelt. Bei dieser Vorbehandlung wird das für die Lösung permeable Gewebe mit einem in der Lösung enthaltenen Farbstoff, insbesondere mit Methylenblau, beladen. Dieser Farbstoff, durch Licht aktiviert, katalysiert photooxidative Reaktionen. Der Konditionierungslösung wird erfindungsgemäss ein Stoff zugegeben, dessen Brechungsindex grösser als jener von Wasser (n = 1,333 bei 25°C) ist. Dank dieses Zusatzstoffes nimmt das kollagenhaltige Gewebe bei der Vorbehandlung eine für das Licht vergrösserte Transparenz an.

Die abhängigen Ansprüche 2 bis 11 beziehen sich auf vorteilhafte Ausführungsformen des erfindungsgemässen Verfahrens und/oder betreffen besondere Aspekte des Verfahrens. Die Ansprüche 11 bis 13 haben Gewebepräparate zum Gegenstand, die nach dem erfindungsgemässen Verfahren behandelt worden sind.

Die photooxidative Behandlung wird gemäss folgendem, drei Schritte umfassenden Verfahren durchgeführt, wobei die Temperatur vorzugsweise bei rund 14 bis 18°C gehalten wird:
1. Vorbehandlung des kollagenhaltigen Gewebes mit der Konditionierungslösung während rund 1 bis 5 Stunden.
2. Belichten des kollagenhaltigen Gewebes während rund 10 bis 50 Stunden.
3. Entfärben des kollagenhaltigen Gewebes sowie Entfernen des Zusatzstoffs durch mehrfaches Waschen mit einer Entfärbungslösung.

Nachfolgend wird die Erfindung anhand der Zeichnungen näher erläutert. Es zeigen:
- Fig. 1: eine Versuchsanordnung zur Durchführung einer photooxidativen Behandlung,
- Fig. 2: Transmission von kollagenhaltigen Geweben in Abhängigkeit von der Gewebedicke,
- Fig. 3: Transmission von Geweben nach Vorbehandlungen mit Konditionierungslösungen, die Glycerin enthalten, und
- Fig. 4: Diagramme zur qualitativen Darstellung der Farbstoffverteilung im Gewebe.

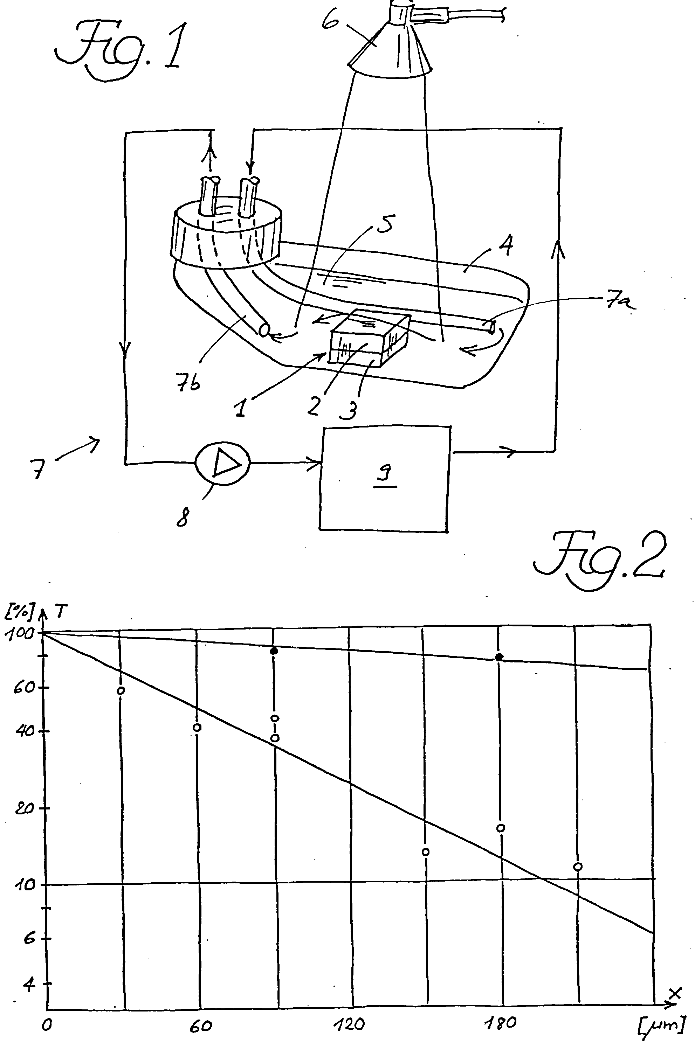

Die Versuchsanordnung der Fig.1 zeigt ein Präparat 1 mit einer Knorpelschicht 2 und einer Knochenschicht 3, das in einem durchsichtigen, geschlossenen Gefäss 4 von einer Lösung 5 um- und überspült wird. Das Präparat 1 wird mit einer Lampe 6 (Kaltlichthalogenlampe, 75 W, 12 V, 10° Öffnungswinkel) bestrahlt. Die Lösung 5 wird im Kreislauf 7 mit einer peristaltischen Pumpe 8 umgewälzt und in einer Kühleinrichtung 9 auf einer vorgegebene Temperatur (16°C) gehalten. Über die Schlauchstücke 7a und 7b wird die Lösung in das Gefäss 4 zu- bzw. aus ihm abgeführt. Die Lösung 5 ist in der Regel die Konditionierungslösung, mit der das Präparat 1 vor der Belichtung vorbehandelt worden ist. Anstelle des Knorpelpräparates 1 kann auch eine Sehne oder ein Band vorgesehen sein.

Die Lösung 5 setzt sich aus einem Phosphatpuffer nach Sφrensen, Glycerin (C₃H₈O₃, Molekulargewicht M = 92 g/mol) und Methylenblau (C₁₆H₁₈ClN₃S.3H₂O, M = 320 g/mol + aq) zusammen. Der Phosphatpuffer nach Sφrensen weist eine Molarität von 1/15 und einen pH-Wert von 7,4 auf; er ist eine Lösung aus reinem Wasser ("Nanopur") und 1,787 g/l Kaliumdihydrogenphosphat (KH₂PO₄, M = 136 g/mol) sowie 9.532 g/l Dinatriumhydrogenphosphat (Na₂HPO₄, M = 178 g/mol). Die Wahl von Methylenblau als Farbstoff ist besonders vorteilhaft, da dieser Stoff nicht toxisch ist (durch verschiedene Anwendungen in der Medizin belegbar). Mit Vorteil sieht man für das Glycerin einen Anteil von rund 30 Vol.-% vor und für das Methylenblau 0,01 Gew.-%.

Präparate 1 werden beispielsweise aus Schultergelenken geschlachteter Rinder entnommen, wobei mittels einer aus der Chirurgie bekannten oszillierenden Säge Knorpelstücke mit Knochen (ca. 10×10×5 mm) aus den Gelenken geschnitten werden. Die Präparate 1 werden in einer Pufferlösung transportiert sowie gelagert und dabei gekühlt gehalten (bei rund 4°C).

Bei der Photooxidation werden Farbstoffmoleküle durch Photonen des eingestrahlten Lichts in einen angeregten Zustand versetzt. In diesem Zustand können sie mit Sauerstoff oder Wasser zu einem Zwischenprodukt reagieren. Während das Zwischenprodukt mit Aminosäureresten des Kollagens (oder anderer Proteine) reagiert, wird der Farbstoff unverändert freigesetzt: er wirkt bei der Photooxidation als Katalysator.

Allerdings finden unter der Einwirkung des Lichts auch irreversible Veränderungen der Farbstoffmoleküle statt, die als Ausbleichung des Farbstoffs in Erscheinung treten. Es muss daher für einen laufenden Nachschub von Farbstoffmolekülen in das Gewebe gesorgt werden. Dies geschieht dadurch, dass das Präparat 1 während der Belichtung ständig mit Konditionierungslösung 5 umspült wird. Über einen Stoffaustausch zwischen dem Gewebe und der Lösung 5 ergibt sich eine teilweise Erneuerung des Farbstoffs im Gewebe, wobei insbesondere ausgebleichter Farbstoff durch intakten ersetzt wird. Gelegentlich-jeweils nach etwa 24 Stunden - muss die gesamte Reaktionslösung durch neue ersetzt werden.

Bei tieferen Temperaturen ist die Photooxidation verlangsamt, und bei höheren Temperaturen kann das kollagenhaltige Gewebe durch die Wärmeeinwirkung geschädigt werden. Durch den Farbstoff wird Licht absorbiert und in Wärme umgewandelt. Die Photooxidation ist eine exotherme Reaktion. Daher muss - um die Behandlung bei einer optimalen Temperatur zwischen rund 14 und 18°C durchführen zu können - die umgewälzte Lösung 5 laufend gekühlt werden.

Bei der Photooxidation werden Wasserstoffionen freigesetzt. Daher muss - um die Behandlung bei einem optimalen pH-Wert zwischen rund 7 und 8 durchführen zu können - eine Pufferlösung verwendet werden (Phosphatpuffer nach Sφrensen).

Bei der Photooxidation wird Sauerstoff verbraucht. Der Gehalt an Sauerstoff, der normalerweise in der Reaktionslösung enthalten ist, reicht in der Regel aus. Einblasen von zusätzlichem Sauerstoff bringt keine Verbesserung des Behandlungsverfahrens.

Mit Vorteil wird Methylenblau mit einer Konzentration von 0,01 Gew.-% verwendet. Bei einer geringeren Konzentration ist die photooxitative Behandlung zu langsam. Bei einer höheren Konzentration wird zu viel Licht in der Reaktionslösung und in den äusseren Bereichen des Gewebes absorbiert, so dass das ganze Gewebe und insbesondere dessen tieferen Bereiche zu wenig Licht empfangen.

Dank des relativ kleinen Molekulargewichts von Methylenblau (M = 320 g/mol) diffundiert dieser Farbstoff gut in und durch das kollagenhaltige Gewebe. Dies bestätigen Untersuchungen an Dünnschnitten.

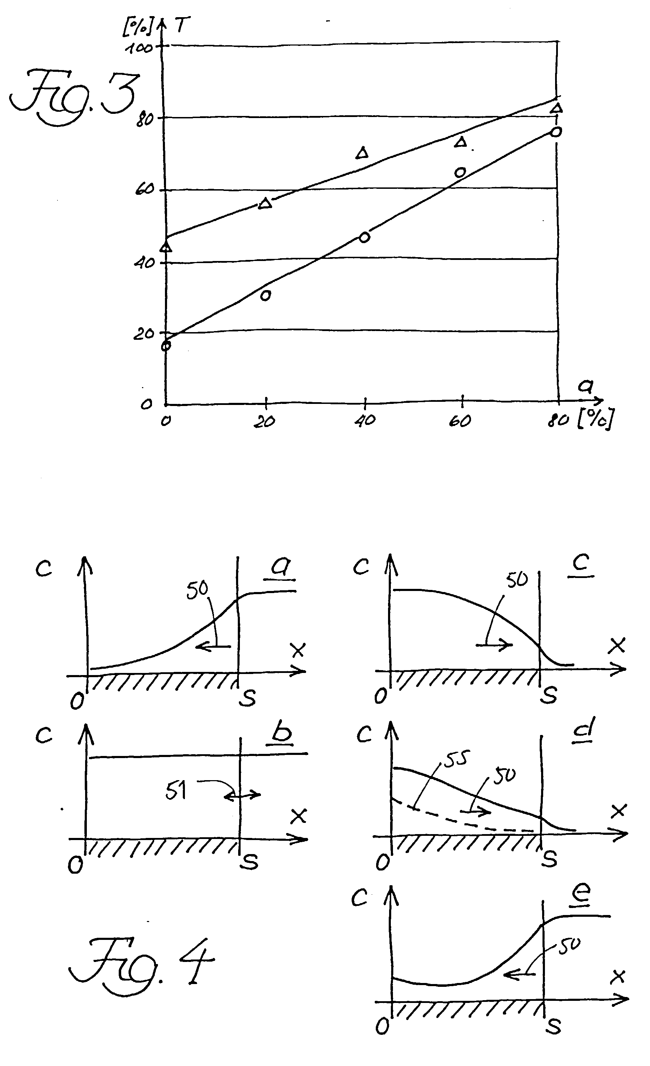

Es ist die Lichtdurchlässigkeit (Transmission) des kollagenhaltigen Gewebes beim Absorptionsmaximum von Methylenblau, nämlich 665 nm, bestimmt worden. Dazu sind Mikrotomschnitte mit Dicken zwischen 30 und 210 µm angefertigt worden. Versuchsergebnisse sind in dem Diagramm der Fig.2 dargestellt. T ist die Transmission und x die Dicke eines ausgemessenen Schnittes. Die Werte für T sind in einem logarithmischen Massstab aufgetragen. Die leeren Kreise geben die Messwerte für Knorpelgewebe an, das gemäss dem bekannten Verfahren vorbehandelt worden ist. Die gefüllten Kreise zeigen die entsprechenden Werte für erfindungsgemäss vorbehandeltes Gewebe, wobei als Zusatzstoff Glycerin mit einem Anteil a von 80 Vol.-% verwendet worden ist.

In Fig.3 ist dargestellt, wie die Transmission T von dem Anteil a an Glycerin abhängt. Die zwei gezeigten Messreihen beziehen sich auf Schichtdicken von 90 µm (Dreiecke) und 180 µm (Kreise).

Wie aus den Diagrammen der Figuren 2 und 3 hervorgeht, ergibt sich durch die erfindungsgemässe Vorbehandlung mit einem Zusatzstoff eine erhebliche Verbesserung der Transparenz des Gewebes. Die Lichteindringtiefe kann um mindestens das Fünffache vergrössert werden. Damit wird die photooxidative Behandlung von Gelenkknorpeln möglich.

Nach der Durchführung der Photooxidation wird das kollagenhaltige Gewebe entfärbt, und gleichzeitig wird der Zusatzstoff entfernt. Dies geschieht durch mehrfaches Waschen mit einer Entfärbungslösung. Mit Vorteil wird als Entfärbungslösung der oben genannte Phosphatpuffer nach SØrensen verwendet. Um die Löslichkeit des Farbstoffs zu vergrössern, kann diesem Phosphatpuffer bis zu 50 Vol-% Ethanol zugemischt sein.

Anhand der Diagramme der Teilfiguren a bis e in Fig.4 sollen noch einige Aspekte der Gewebeeinfärbung erläutert werden. Die Diagramme stellen die Farbstoffkonzentration c als Funktion von x dar. Durch x ist der Abstand von der Knochenoberfläche (x = 0) gegeben. Über das Intervall 0 < x < s erstreckt sich das Knorpelgewebe. Im Gebiet x > s befindet sich die Reaktionslösung bzw. die Entfärbungslösung.

Fig.4a illustriert die Anfangsphase des Einfärbens während der Vorbehandlung. Der Pfeil 50 deutet den auf Diffusion beruhenden Farbstofftransport an. Nach erfolgter Vorbehandlung sollte ein Gleichgewicht mit einer homogenen Farbstoffverteilung vorliegen. Dies ist in Fig.4b gezeigt. Der Doppelpfeil 51 deutet an, dass weiterhin ein Stoffaustausch über die Gewebeoberfläche bei x = s stattfindet, durch welchen sich ein Ersatz von ausgebleichten Farbstoffmolekülen im Gewebe ergibt.

Das Entfärben nach der Photooxidation stellen die Figuren 4c und 4d dar. Da während des Entfärbens die Farbstoffkonzentration in den tieferen Bereichen des Gewebes einige Zeit noch relativ hoch ist, kann die Belichtung mit Vorteil auch während dieser Verfahrensphase fortgesetzt werden, und zwar bis zu einem Zeitpunkt, da die Konzentration c beispielsweise den gestrichelt gezeichneten Kurvenverlauf 55 annimmt. Dies ist vorteilhaft, da die Photooxidation zuvor in den tieferen Bereichen weit weniger wirksam als in den oberen Bereichen gewesen ist.

Die Lehre, auch während des Entfärbens die Belichtung fortzusetzen, lässt sich zu einer vorteilhaften Verfahrensvariante weiterentwickeln: Die Belichtung soll intermittierend vorgenommen werden, nämlich derart, dass während Belichtungsphasen das Knorpelgewebe gleichzeitig entfärbt wird und dass während Zwischenphasen das Knorpelgewebe ohne Belichtung mit Konditionierungslösung behandelt und erneut eingefärbt wird. Eine erneute Einfärbung des Gewebes ist durch das Diagramm der Fig.4e illustriert.

Ist eine Verwendung des photooxidativ behandelten Knorpelgewebes als Implantat vorgesehen, so soll die Behandlung des Gewebes in dem Ausmass durchgeführt werden, dass sich die Kollagenfasern in der Weise verändern, dass bei einem Patienten immunologische Abwehrreaktionen unterbleiben oder nur schwach ausgeprägt sind. Ausserdem soll durch die photooxidative Behandlung das Knorpelgewebe eine grössere mechanische Beanspruchbarkeit gewinnen.

Bei der Belichtung von Knorpelpräparaten kann die Belichtung wirkungsvoll nur von der einen Seite vorgenommen werden. Bei der erfindungsgemässen Behandlung von Bändern und Sehnen ist eine Belichtung von beiden oder allen Seiten möglich.

## Patentansprüche

1. Verfahren zu einer photooxidativen Behandlung von kollagenhaltigen Geweben, bei welchem Verfahren das Gewebe vor einer Bestrahlung mit Licht mittels einer wässrigen Konditionierungslösung vorbehandelt wird, so dass das für die Lösung permeable Gewebe mit einem in der Lösung enthaltenen Farbstoff, insbesondere Methylenblau, beladen wird, wobei dieser Farbstoff durch Licht aktiviert photooxidative Reaktionen katalysiert,
dadurch gekennzeichnet, dass in die Lösung ein Zusatzstoff zugegeben wird, dessen Brechungsindex grösser als jener von Wasser - nämlich 1,333 bei 25°C - ist und mit dem das Gewebe bei der Vorbehandlung eine für das Licht vergrösserte Transparenz annimmt.

2. Verfahren nach Anspruch 1, dadurch gekennzeichnet, dass der zusätzliche Stoff einen Brechungsindex grösser als 1,4 aufweist und dass dieser Stoff insbesondere Glycerin ist.

3. Verfahren nach Anspruch 1 oder 2, dadurch gekennzeichnet, dass in Anwesenheit von Sauerstoff und unter der katalytischen Wirkung des Farbstoffs Kollagenfasern in der Weise photooxidativ verändert werden, dass bei Verwendung des behandelten Gewebes als Implantat in einem Patienten immunologische Abwehrreaktionen unterbleiben oder nur schwach ausgeprägt sind.

4. Verfahren nach Anspruch 3, dadurch gekennzeichnet, dass die Kollagenfasern durch die photooxidative Behandlung teilweise miteinander vernetzt werden, so dass das Gewebe eine grössere mechanische Beanspruchbarkeit gewinnt.

5. Verfahren nach einem der Ansprüche 1 bis 4, dadurch gekennzeichnet, dass folgende drei Schritte, vorzugsweise bei 14 bis 18°C, durchgeführt werden:
1. Vorbehandlung des Gewebes mit der Konditionierungslösung während 1 bis 5 Stunden,
2. Belichten des Gewebes während 10 bis 50 Stunden, und
3. Entfärben des kollagenhaltigen Gewebes sowie Entfernen des Zusatzstoffs durch mehrfaches Waschen mit einer Entfärbungslösung.

6. Verfahren nach Anspruch 5, dadurch gekennzeichnet, dass die Konditionierungslösung ein Gemisch aus einem Phosphatpuffers und Glycerin ist, wobei ein Phosphatpuffer nach Sørensen verwendet wird, der eine Molarität von 1/15 und einen pH-Wert von 7,4 aufweist, der Anteil an Glycerin 30 Vol-% beträgt und der Anteil an Methylenblau 0,01 Gew-% beträgt.

7. Verfahren nach Anspruch 5 oder 6, dadurch gekennzeichnet, dass die Entfärbungslösung ein Phosphatpuffer ist, dem 0 bis 50 Vol-% Ethanol zugemischt ist, wobei der Phosphatpuffer ein Phosphatpuffer nach Sφrensen ist, der eine Molarität von 1/15 und einen pH-Wert von 7,4 aufweist.

8. Verfahren nach einem der Ansprüche 1 bis 7, dadurch gekennzeichnet, dass während der ganzen Belichtungsphase oder während eines grossen Teils der Belichtungsphase das Gewebe mit sauerstoffhaltiger Konditionierungslösung umspült wird.

9. Verfahren nach Anspruch 8, dadurch gekennzeichnet, dass die Konditionierungslösung mit einer Pumpe in einem Kreislauf geführt wird und dass mittels einer in diesem Kreislauf vorgesehenen Kühleinrichtung eine vorgegebene Temperatur der umgewälzten Lösung eingestellt wird.

10. Verfahren nach Anspruch 8 oder 9, dadurch gekennzeichnet, dass während einer die Belichtung abschliessenden Phase das Gewebe mit sauerstoffhaltiger Entfärbungslösung umspült wird.

11. Verfahren nach Anspruch 5, dadurch gekennzeichnet, dass die Belichtung intermittierend vorgenommen wird, dass während Belichtungsphasen das Gewebe mit sauerstoffhaltiger Entfärbungslösung umspült wird und dass während Zwischenphasen das Gewebe ohne Belichtung mit Konditionierungslösung behandelt wird.

12. Knorpelpräparat (1) mit einer Knochenschicht (3) und einer Knorpelschicht (2), die mit dem Verfahren gemäss einem der Ansprüche 1 bis 11 behandelt worden ist.

13. Kollagenhaltiges Gewebe, das aus einer Sehne oder einem Band gewonnen worden ist und das mit dem Verfahren gemäss einem der Ansprüche 1 bis 11 behandelt worden ist.

## Claims

1. A method of photo-oxidative treatment of tissues containing collagen, in which method the tissue is pre-treated with an aqueous conditioning solution prior to irradiation with light, so that the tissue permeable for the solution is'loaded with a dye contained in the solution, in particular methylene blue, wherein this dye catalyses photo-oxidative reactions activated by light,
characterized in that, an additive is added to the solution, the refractive index of which is greater than that of water - namely 1,333 at 25° C-and with which the tissue takes on a greater transparency for the light during the pre-treatment.

2. A method in accordance with claim 1, characterized in that, the additive has a refractive index greater than 1,4 and in that this substance is in particular glycerine.

3. A method in accordance with claim 1 or claim 2, characterized in that in the presence of oxygen and under the catalytic effect of the dye, collagenous fibres are photo-oxidatively altered in such a manner that if the treated tissue is used as an implant in a patient, immunological defence reactions do not occur or are only slight.

4. A method in accordance with claim 3, characterized in that the collagenous fibres are partially crosslinked with one another by the photo-oxidative treatment, so that the tissue gains a greater ability to withstand mechanical loading.

5. A method in accordance with one of the claims 1 to 4, characterized in that the following three steps are carried out, preferably at 14 to 18° C:
1. Pre-treatment of the tissue with the conditioning solution for 1 to 5 hours,
2. Exposure of the tissue for 10 to 50 hours, and
3.Discolouring of the collagenous tissue and also removal of the additive by means of repeated washing with a decolourising solution.

6. A method in accordance with claim 5, characterized in that the conditioning solution is a mixture of a phosphate buffer and glycerine, wherein a phosphate buffer in accordance with Sorensen is used, which has a molar concentration of 1/15 and a pH value of 7.4, in which the proportion of glycerine amounts to 30 percent by volume and the proportion of methylene blue amounts to 0.01 percent by weight.

7. A method in accordance with claim 5 or claim 6, characterized in that the decolourising solution is a phosphate buffer, to which 0 to 50 percent by volume ethanol is added, wherein the phosphate buffer is a phosphate buffer in accordance with Sorensen, which has a molar concentration of 1/15 and a pH value of 7.4.

8. A method in accordance with one of the claims 1 to 7, characterized in that during the entire exposure phase or during a greater part of the exposure phase the tissue is rinsed in an oxygen containing conditioning solution.

9. A method in accordance with claim 8, characterized in that the conditioning solution is moved in a circuit by a pump and that the
circulated solution is adjusted to a pre-given temperature by means of a cooling apparatus provided in this circuit.

10. A method in accordance with claim 8 or claim 9, characterized in that, during a phase
concluding the exposure the tissue is rinsed with an oxygen containing decolourising solution.

11. A method in accordance with claim 5, characterized in that, the exposure is carried out intermittently, in that during exposure phases the tissue is rinsed with an oxygen containing decolourising solution and in that during intermediate phases the tissue is treated with conditioning solution without exposure.

12. A cartilage preparation (1) with a bone layer (3) and a cartilage layer (2), which is treated with the method in accordance with one of the claims 1 to 11.

13. Collagen containing tissue, which is won from a tendon or a ligament and which has been treated with the method according to one of the claims 1 to 11.

## Revendications

1. Procédé de traitement photooxydatif de tissus contenant du collagène, procédé dans lequel le tissu, avant une irradiation avec de la lumière, est traité préalablement au moyen d'une solution de conditionnement aqueuse de telle sorte que le tissu perméable à la solution est chargé avec un colorant contenu dans la solution, notamment en bleu de méthylène, où ce colorant, activé par la lumière, catalyse des réactions photooxydatives, caractérisé en ce qu'il est ajouté dans la solution un additif dont l'indice de réfraction est plus grand que celui de l'eau - à savoir 1,333 à 25°C-et avec lequel le tissu, lors du traitement préalable, atteint une transparence plus grande pour la lumière.

2. Procédé selon la revendication 1, caractérisé en ce que le produit additionnel présente un indice de réfraction supérieur à 1,4, et que ce produit est notamment de la glycérine.

3. Procédé selon la revendication 1 ou 2, caractérisé en ce qu'en présence d'oxygène et sous l'effet catalytique du colorant, des fibres de collagène sont modifiées d'une manière photooxydative de telle sorte que lors de l'utilisation du tissu traité comme implant, dans un patient des réactions de défense immunologiques n'ont pas lieu ou sont seulement peu prononcées.

4. Procédé selon la revendication 3, caractérisé en ce que les fibres de collagène sont partiellement réticulées par le traitement photooxydatif les unes avec les autres de telle sorte que le tissu peut être sollicité mécaniquement plus fortement.

5. Procédé selon l'une des revendications 1 à 4, caractérisé en ce que les trois étapes suivantes sont exécutées, de préférence entre 14 et 18°C :
1. traitement préalable du tissu avec la solution de conditionnement pendant 1 à 5 heures.
2. exposition à la lumière du tissu pendant 10 à 50 heures et
3. décoloration du tissu contenant du collagène et retrait de l'additif par lavage répété avec une solution de décoloration.

6. Procédé selon la revendication 5, caractérisé en ce que la solution de conditionnement est un mélange d'un tampon phosphate et d'une glycérine, où est utilisé un tampon phosphate selon Sørensen, qui a une concentration molaire de 1/15 et une valeur pH de 7,4, où la part en glycérine est de 30% en volume et la part en bleu de méthylène de 0,01% en poids.

7. Procédé selon la revendication 5 ou 6, caractérisé en ce que la solution de décoloration est un tampon phosphate auquel on ajoute 0 à 50% en volume d'éthanol, le tampon phosphate étant un tampon phosphate selon Sørensen qui présente une concentration molaire de 1/15 et une valeur pH de 7,4.

8. Procédé selon l'une des revendications 1 à 7, caractérisé en ce que pendant toute la durée d'exposition à la lumière ou pendant une grande partie de la phase d'exposition à la lumière, le tissu est lavé avec une solution de conditionnement contenant de l'oxygène.

9. Procédé selon la revendication 8, caractérisé en ce que la solution de conditionnement est conduite en circuit au moyen d'une pompe et en ce qu'il est réglé, au moyen d'un dispositif de refroidissement prévu dans ce circuit, une température prédéterminée de la solution en circulation.

10. Procédé selon la revendication 8 ou 9, caractérisé en ce que pendant une phase terminant l'exposition à la lumière, le tissu est lavé avec une solution de décoloration contenant de l'oxygène.

11. Procédé selon la revendication 5, caractérisé en ce que l'exposition à la lumière est exécutée par intermittence, en ce que pendant les phases d'exposition à la lumière, le tissu est lavé avec une solution décolorante contenant de l'oxygène et en ce que pendant les phases intermédiaires, le tissu est traité sans exposition à la lumière avec la solution de conditionnement.

12. Préparation de cartilage (1) avec une couche d'os (3) et une couche de cartilage (2) qui a été traitée selon le procédé conformément à l'une des revendications 1 à 11.

13. Tissu contenant du collagène qui a été obtenu à partir d'un tendon ou d'un ligament et qui a été traité avec le procédé selon l'une des revendications 1 à 11.
